# EUROPÄISCHE PATENTSCHRIFT

(11) **EP 1 769 250 B1**
(45) Veröffentlichungstag und Bekanntmachung des Hinweises auf die Patenterteilung: **03.06.2009**
(21) Anmeldenummer: 06706415.4
(22) Anmeldetag: 25.01.2006
(51) Int. Cl.: G01N 33/74, C07K 16/18, C07K 16/28

(54) **IMMUNOASSAY ZUR BESTIMMUNG DER FREISETZUNG VON NEUROTENSIN IN DIE ZIRKULATION**
IMMUNOASSAY FOR DETERMINING THE RELEASE OF NEUROTENSIN INTO THE CIRCULATION
ESSAI IMMUNOLOGIQUE POUR DETERMINER LA LIBERATION DE NEUROTENSINE DANS LA CIRCULATION

(30) Priorität: 26.01.2005 DE 102005003687
(43) Veröffentlichungstag der Anmeldung: 04.04.2007
(73) Patentinhaber: SphingoTec GmbH, 16556 Borgsdorf (DE)
(72) Erfinder: ERNST, Andrea, 16761 Hennigsdorf (DE)
(74) Vertreter: Andrae, Steffen
(86) Internationale Anmeldenummer: PCT/EP2006/000659
(87) Internationale Veröffentlichungsnummer: WO 2006/079528

(56) Entgegenhaltungen:
- BIDARD JEAN-NOEL ET AL: "Immunological and biochemical characterization of processing products from the neurotensin/neuromedin N precursor in the rat medullary thyroid carcinoma 6-23 cell line" BIOCHEMICAL JOURNAL, Bd. 291, Nr. 1, 1993, Seiten 225-233, XP002381242 ISSN: 0264-6021
- EKLUND S ET AL: "EFFECTS OF CHOLERA TOXIN ESCHERICHIA-COLI HEAT STABLE TOXIN AND SODIUM DEOXYCHOLATE ON NEUROTENSIN RELEASE FROM THE ILEUM IN-VIVO" REGULATORY PEPTIDES, Bd. 26, Nr. 3, 1989, Seiten 241-252, XP009066470 ISSN: 0167-0115

## Beschreibung

Die vorliegende Erfindung betrifft ein für die Routinediagnostik geeignetes Verfahren zur Bestimmung der Freisetzung des Peptids Neurotensin in die Zirkulation.

Dabei soll vorausschickend angemerkt werden, dass in der vorliegenden Beschreibung Begriffe wie "Diagnostik" bzw. "Routinediagnostik" aus Gründen der Vereinfachung in der Regel in einem umfassenden Sinne verwendet werden, der die Bestimmung der gemessenen Biomoleküle (Peptide) nicht nur für diagnostische Zwecke im engeren Sinne abdecken soll, sondern auch für andere Zwecke wie z.B. zur Bestimmung des metabolischen Zustands eines Probanden vor Beginn einer Therapie oder einer Untersuchung zu diagnostischen Zwecken oder zur Beobachtung und Überwachung eines Patienten über längere Zeiträume, z.B. zur Kontrolle eines Krankheitsverlaufs oder von Therapiemaßnahmen. Auch Bestimmungen zur Forschungszwecken, z.B. im Zusammenhang mit der Entwicklung von Pharmazeutika, z.B. Neurotensinrezeptor-Agonisten, oder von Produkten für die Ernährung, sollen unter den Begriff "Diagnostik" subsummiert werden, soweit das nicht im Einzelfall aufgrund der diskutierten spezielleren Zusammenhänge ausgeschlossen ist.

Außerdem gilt, dass auch wenn die spezielleren Ausführungen zur Erfindung in der vorliegenden Anmeldung insbesondere die Humanmedizin betreffen, Anwendungen auf dem Gebiet der Tiermedizin oder Tierzucht grundsätzlich ebenfalls eingeschlossen sein sollen. So ergibt sich für den Fachmann die Übertragbarkeit der meisten Ausführungen zur vorliegenden Erfindung auf nicht-menschliche Säugetiere, ohne dass umfangreiche Erläuterungen nötig sind. Die analoge Anwendbarkeit des für die Humanmedizin näher beschriebenen Verfahrens auf die Tiermedizin folgt z.B. aus der Bekanntheit der entsprechenden Peptidsequenzen für viele andere Säugetiere, und neben tiermedizinischen Anwendungen sind auch Anwendungen denkbar, die eher die Säugetierzucht betreffen, z.B. als Monitoring des Fütterungszustands und der Futterverwertung von Nutztieren.

Neurotensin ist ein in verschiedenen Geweben und der Zirkulation von verschiedenen Säugetieren, einschließlich des Menschen, vorkommendes Tridecapeptid, dessen Primärstruktur bei allen Säugetieren identisch zu sein scheint. Es weist die Aminosäuresequenz auf, die im Sequenzprotokoll als SEQ ID NO:2 aufgeführt ist.

Neurotensin wird, wie die meisten biologisch aktiven Peptide (5; Literaturangaben in Form von Zahlen in Klammern beziehen sich auf die beigefügte Literaturliste), durch enzymatische Prozessierung aus einem Vorläufermolekül gebildet, das als Preproneurotensin (mit Signalsequenz) bzw. Proneurotensin (PNT; ohne Signalsequenz) bezeichnet wird. Das Preproneurotensin/Proneurotensin der Säuger enthält neben dem Neurotensin (NT) ein weiteres biologisch aktives Peptid, das Hexapeptid Neuromedin N (NMN; vgl. SEQ ID NO:3). Neurotensin-Vorläuferpeptide wurden u.a. aus Hunde-, Rinder- und Nagetier-Geweben und schließlich auch aus humanen Geweben isoliert. Während die reifen Peptide NT und NMN für verschiedene Säugetiere eine identische Aminosäuresequenz aufweisen, unterscheiden sich die Vorläufermoleküle, d.h. die Prepro- bzw. Proneurotensine, von verschiedenen Säugetier-Spezies bezüglich einer Anzahl von Aminosäuren. So weist das Preproneurotensin der Ratte oder des Rinds z.B. nur 169 Aminosäuren auf, wohingegen das humane Preproneurotensin 170 Aminosäuren aufweist (17).

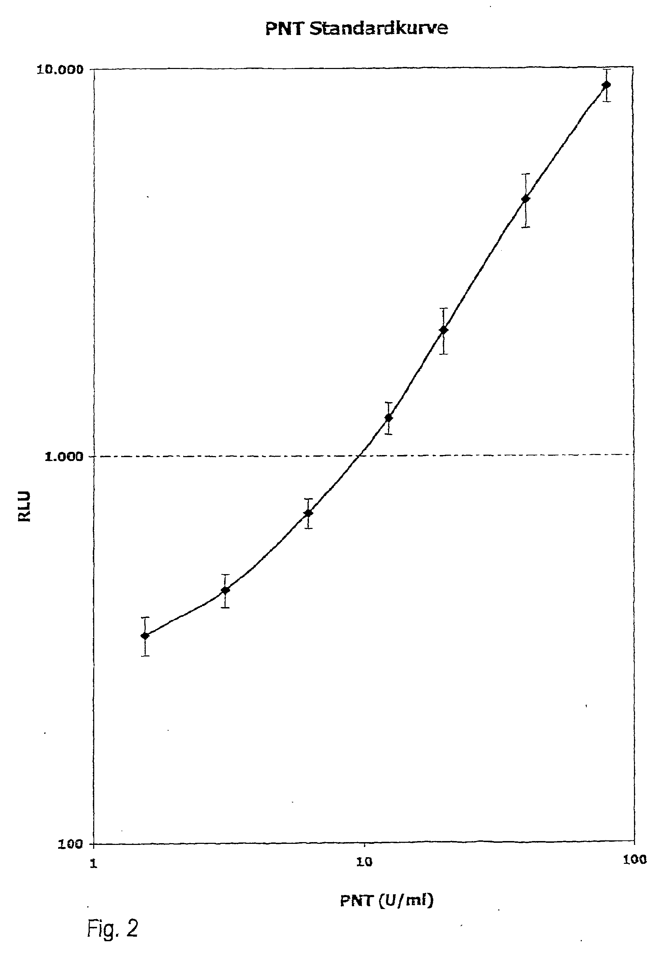

Die Nukleotid- und Aminosäure-Sequenzen des humanen Preproneurotensins sind seit einigen Jahren bekannt (SEQ ID NO:1; vgl. auch Patent US 6,274,720 B1), genau wie die aus Hund (9), Ratte (17) und Rind (17; vgl. auch die Gegenüberstellung verschiedener Preproneurotensinsequenzen in Figur 2 des o.g. US-Patents). Die Aminosäuren 1 bis 23 des Preproneurotensins (SEQ ID NO:1) stellen die sog. Signalsequenz dar.

Die Expression von Proneurotensin erfolgt vorrangig im zentralen Nervensystem (ZNS) und in speziellen enteroendocrinen Zellen, den sogenannten N-Zellen im distalen Dünndarm sowie in Nervenfasern, die den Darmtrakt durchziehen (13). PNT bzw. NT wurden aber auch in den Nebennieren und im Herzgewebe nachgewiesen (24; 23).

Das aus dem Proneurotensin gebildete biologisch aktive Neurotensin (NT) wurde in verschiedenen Säugetier-Geweben nachgewiesen und im Zusammenhang mit zahlreichen unterschiedlichen Funktionen diskutiert. So spielt Neurotensin im Gehirn eine große Rolle als Neurotransmitter und hat analgetische und thermoregulatorische Effekte (6; 8). Des weiteren reguliert NT die Freisetzung der Neurotransmitter Acetylcholin und Glutamat (19; 18), stimuliert die Sekretion von Hormonen der Hypophyse (29) und beeinflusst die Dopamin-Neurotransmission (18), was einen Zusammenhang zur Parkinson'schen Erkrankung nahe legt (28).

Die Sekretion von Neurotensin im Gastrointestinaltrakt wird durch Nahrungsaufnahme stimuliert (15). Dabei kommt es bereits kurz nach dem Essen zu einem signifikanten NT-Anstieg, der über mehrere Stunden erhöht bleibt (14). Es konnte gezeigt werden, dass die Aufnahme von Fett, im Vergleich mit Eiweiß und Glucose, den stärksten Einfluss auf die NT-Produktion hatte (27). Neurotensin stellt offensichtlich eine Art "Sättigungsfaktor" dar, da die Ausschüttung von NT zur Zügelung des Appetits und somit zur Reduktion der Nahrungsaufnahme führt (34). Bei adipösen (fettleibigen) Patienten ist die Plasma-Konzentration des NT im Vergleich zu normalgewichtigen Personen reduziert, was vermutlich zu einem gesteigerten Appetit und einer erhöhten Aufnahme von Nahrung führt (35; 4). Interessanterweise zeigten auch Raucher einen signifikant höheren Anstieg der NT-Konzentration nach Nahrungsaufnahme als Nichtraucher (26). Dies lässt vermuten, dass eine veränderte Neurotensinausschüttung eine mögliche Ursache des gesteigerten Appetits und der häufig zu beobachtenden Gewichtszunahme von ehemaligen Rauchern nach Einstellung des Rauchens ist.

Im Gastrointestinaltrakt stimuliert Neurotensin die Sekretion von Pankreashormonen wie Insulin und Glucagon, inhibiert die Sekretion von Magensäure und stimuliert die Dickdarmmotilität (33). NT fördert das Wachstum von gastrointestinalem Gewebe wie Pankreas-, Dick- und Dünndarmgewebe (12; 36; 10), was die Hypothese nahe legt, dass NT zur Entstehung von Tumoren in diesen Geweben beiträgt.

Die Funktion des zweiten aus dem gleichen Vorläuferpeptid gebildeten Peptids Neuromedin N (SEQ ID NO:3) ist weniger gut charakterisiert. Vermutlich hat es ähnliche Effekte wie das Neurotensin, da beide Ähnlichkeiten in ihrer Aminosäuresequenz aufweisen und an die gleichen Rezeptoren binden (33).

Durch Untersuchung von verschiedenen Gewebeextrakten unter Verwendung von Radioimmunoassays unter Verwendung von Antikörpern, die für freie C-Termini von NT oder NMN spezifisch sind und daher nicht proteolytisch prozessierte Proneurotensin-Formen ohne derartige freigelegte COOH-Temini nicht erkennen, teilweise in Kombination mit HPLC-Techniken zur Auftrennung der Fraktionen nach ihrer Molekülgröße, wurde festgestellt, dass neben NT und NMN bei der Prozessierung von PNT in gewebeabhängiger Weise auch unvollständig gespaltene, sog. große ("large") NT- und/oder NMN-Peptide gebildet werden können (7; 13; 25). Antikörper, die Neurotensin erkennen bzw. damit kreuzreagieren, sind offenbart in (37; 38)

Zahlreiche Untersuchungen befassen sich mit der Bildung bzw. dem Vorkommen von Neurotensin bei verschiedenen Krankheiten, wobei diese Untersuchungen in erster Linie mit Gewebeproben und Gewebeextrakten durchgeführt wurden. Von Untersuchungen, die auf Messungen von Neurotensin in Blutproben von Erkrankten aufbauen, ist z.B. zu nennen, dass die Konzentration von Neurotensin im Plasma von Patienten mit Morbus Parkinson erhöht gefunden wurde (30). Bei Zöliakiepatienten, die eine Unverträglichkeit gegenüber Getreidegluten aufweisen, wurde im Plasma im Vergleich zu Kontrollpersonen im nüchternen Zustand eine erhöhte Neurotensin-Konzentration nachgewiesen (2). Die Plasmakonzentrationen von Neurotensin bei Patienten mit einer Pankreatitis sind nach Einnahme einer Mahlzeit im Vergleich zu gesunden Kontrollpersonen erhöht (22). Bei Schilddrüsenerkrankungen wurden im Plasma von Patienten mit zentraler (oder sekundärer) Schilddüsenunterfunktion signifikant niedrigere Neurotensin-Konzentrationen festgestellt (31). Die sekundäre Schilddrüsenunterfunktion ist bedingt durch eine Störung im Bereich der Hypophyse, welche die Ausschüttung von Schilddrüsenhormonen steuert. Dagegen konnten im Blut von Patienten mit einer peripheren (oder primären) Schilddrüsenunter- bzw. Schilddrüsenüberfunktion im Vergleich mit Kontrollen signifikant höhere Neurotensin-Spiegel (31) festgestellt werden. Bei der primären Schilddrüsenunterfunktion liegt die Ursache in einer Funktionsstörung der Schilddrüse selbst begründet. Patienten mit einer cystischen Fibrose (Mukoviszidose) weisen sowohl vor als auch nach einer Nahrungsaufnahme höhere Neurotensin-Konzentrationen im Plasma auf als gesunde Kontrollpersonen (16, 1; 21).

Zur Bestimmung der Neurotensin-Spiegel in Blut wurden ausnahmslos Assays vom Typ Radioimmunoassay eingesetzt, bei denen die Kompetition des zu bestimmenden Neurotensins in der Probe mit einem zugesetzten markierten Neurotensin oder Neurotensinfragment um die Bindungsstellen eines einzigen Antikörpers bestimmt wird, der eine spezifische Bindung mit Neurotensin bzw. bestimmten Teilsequenzen des freien Neurotensins eingeht, z.B. mit einem freien C-Terminus des Neurotensins. Messungen der NMN-Spiegel wurden auf ähnliche Weise durchgeführt.

Die obigen und ähnliche Ergebnisse zum Vorkommen von Neurotensin bei bestimmten Erkrankungen bzw. die nachgewiesene Stimulierbarkeit der Neurotensinbildung in Abhängigkeit von der Nahrungsaufnahme, die sich außerdem für bestimmte Krankheitszustände als signifikant unterschiedlich erwies, macht die Bestimmung von Neurotensin in Blutproben an sich zu einem vielversprechenden und klinisch vielseitig anwendbaren diagnostischen Hilfsmittel. So erscheint eine solche Bestimmung z.B. im Rahmen der Routineuntersuchung von Patienten, zu ihrer Überwachung während ihrer klinischen Betreuung, zur Kontrolle ihrer Fähigkeit, Nahrung zu verwerten, im Sinne eines Monitorings der Darmfunktionen, oder als therapiebegleitende Maßnahme zur Kontrolle von Therapieerfolgen, wie auch in anderen Zusammenhängen, die sich aus der o.g. Diskussion der wissenschaftlichen Literatur ergeben, einsetzbar.

Neurotensinbestimmungen in Blutproben haben jedoch bisher keinen Eingang in die medizinische Routinediagnostik gefunden. Ein erster wesentlicher Grund dafür liegt in einer für Routinemessungen zu geringen Stabilität der Neurotensinkonzentrationen in Blutproben ex vivo bei Umgebungstemperatur (20) (Halbwertszeit von nur 3 bis 4 h), die in Kombination mit bestehenden Laborlogistiken (von Probenentnahme, Plasmagewinnung, Transport ins Labor bis zur Durchführung von entsprechenden Labortesten) bislang die Verwendung von Neurotensin in der Routinediagnostik verhinderte. Ein zweiter Grund liegt in der bekannten kurzen Halbswertzeit von nur 2 bis 6 min für Neurotensin im Blut in vivo, die auf eine schnelle und effektive Bindung an Neurotensinrezeptoren und/oder einen Abbau des Neurotensins zurück geführt wird (3) und eine Messung mit einer potentiell schwierig einzuschätzenden Zeitabhängigkeit belastet. Momentan messbare NT-Spiegel in Blutproben stellen kein Maß für die gesamte auf einen stimulierenden Reiz, z.B. eine Nahrungszufuhr, bis zum Messzeitpunkt erfolgte Biosynthese und Freisetzung von NT in die Zirkulation dar, sondern repräsentieren eher einen zufälligen Durchgangszustand, bei dem die NT-Eliminierung durch Bindung an Rezeptoren und durch Abbau die Informationen zur Biosynthese und Freisetzung in die Zirkulation massiv verwässert.

Es ist Aufgabe der vorliegenden Erfindung, eine routinetaugliche Möglichkeit zur Bestimmung der Neurotensin-Freisetzung in Proben vom Säugetier-Probanden, insbesondere menschlichen Patienten, zu schaffen, die es ermöglicht, das oben skizzierte Potential einer Bestimmung der Neurotensinfreisetzung für die medizinische Diagnostik auszunutzen und für die medizinisch/klinische Routine umzusetzen.

Diese Aufgabe wird durch ein immundiagnostisches Bestimmungsverfahren zur Bestimmung der Freisetzung von Neurotensin in die Säugetier-Zirkulation gelöst, das in seiner allgemeinsten Form gemäß Anspruch 1 dadurch gekennzeichnet ist, dass man in einer Serum- oder Plasmaprobe eines Säugetier-Probanden selektiv eine Immunreaktivität des N-terminalen Teils eines Säugetier-Proneurotensins (PNT-Immunreaktivität) bestimmt, die keine Neurotensin- oder Neuromedin N-Immunreaktivität ist.

Vorteilhafte Ausgestaltungen eines solchen Verfahrens werden durch die Ansprüche 2 bis 16 in Verbindung mit den Ausführungen zu bevorzugten Ausführungsformen in der Beschreibung wiedergegeben.

Nachfolgend werden die der Erfindung zugrunde liegenden neuen Erkenntnisse, und die daraus abgeleiteten erfindungsgemäßen Anwendungen, unter Bezugnahme auf konkrete Versuchsergebnisse und Figuren noch näher erläutert.

Dabei bedeutet "Immunreaktivität des N-terminalen Teils eines Säugetier-Proneurotensins" eine Immunreaktivität, die im Bereich von der Aminosäure in Position 24 (Position 1 nach dem Signalpeptid) bis zur drittletzten Aminosäure vor der ersten Aminosäure des Neuromedin N (Lys) des jeweiligen Säugetier-Preproneurotensins lokalisiert ist. Der angesprochene Bereich umfaßt in allen bekannten Fällen mindestens die jeweiligen Aminosäuren 24 bis 139 bzw. 140, d.h. eine Sequenz, wie sie für das humane Peptide in SEQ ID NO:6 gezeigt ist.

In den Figuren zeigen:
- Figur 1:: Ergebnisse der Messung der *ex vivo* Stabilität der Immunreaktivität, die N-terminalen Sequenzen des humanen PNT zuzuordnen ist, in aus Probandenblut gewonnenen Serum-, EDTA-Plasma- und Heparin-Plas- ma-Proben bei Raumtemperatur über Zeiträume von bis zu 7 Tagen;
- Figur 2:: Die Standardkurve eines PNT-Sandwichassays, wie er im experimentellen Teil näher beschrieben wird;
- Figur 3:: Die Ergebnisse der Messung der PNT-Immunreaktivi- tät in Plasma bei normal ernährten Normalpersonen und künstlich ernährten Patienten mittels des beschriebenen Sandwich-Assays;
- Figur 4:: Die Ergebnisse der Messung zeitlicher Veränderun- gen der PNT-Immunreaktivität im Plasma von normal ernährten Normalpersonen in Abhängigkeit von der Nahrungszufuhr mittels des beschriebenen Sandwich- Assays;
- Figur 5:: Die Ergebnisse der Messung der PNT-Immunreaktivi- tät in Plasma bei normal ernährten Normalpersonen, Patienten mit chronisch-entzündlichen Darmerkran- kungen (Morbus Crohn/Colitis ulcerosa) und Hepati- tis-Patienten mittels des beschriebenen Sandwich- Assays.

Zur Überprüfung der Frage, ob bei einer Bestimmung einer Immunreaktivität, die dem N-terminalen Teil des humanen Proneurotensins zuzuordnen ist, und zwar einem Teil, der weder NMN noch NT enthält und bei der proteolytischen Prozessierung unter Bildung von freiem NMN bzw. NT abgespalten wird und dann eine Art Abfallprodukt darstellt, in einer Blutprobe bzw. Serum- oder Plasmaprobe reproduzierbar Ergebnisse erhalten werden können, die den Literaturangaben zum Auftreten von NT entsprechen, wurde ein für diesen N-Terminus des PNT spezifischer Sandwich-Assay entwickelt, wie er im experimentellen Teil näher beschrieben wird. Dieser Assay erkennt spezifisch nur N-terminale Peptide des humanen PNT, die sowohl die Aminosäuren 67 bis 85 als auch die Aminosäuren 121 bis 140 des humanen Preproneurotensins enthalten.

Messungen von humanen Blutproben mit diesem Assay, die nachfolgend in näheren Einzelheiten beschrieben werden, zeigten, dass damit PNT-Immunreaktivitäten gemessen werden, die nach Auftreten und Dynamik die Bildung/Freisetzung von NT widerspiegeln.

Bei der wiederholten Messung von aus derartigen Blutproben gewonnenen Serum- und Plasmaproben bei einer Lagerung bei Raumtemperatur über längere Zeiräume zeigte sich ferner, dass die Stabilität der PNT-Immunreaktivität in Plasma ex vivo überraschend hoch ist. Da selbst nach 7 Tagen noch kein erkennbarer Verlust an bestimmbarer Immunreaktivität feststellbar war, liegt ihre Halbwertszeit bei Raumtemperatur bei weit mehr als 7 Tagen und damit weit über der ex vivo Stabilität des reifen Neurotensins (vgl. 3) bzw. derjenigen eines der großen ("large"), unvollständig prozessierten Proneurotensinpeptide.

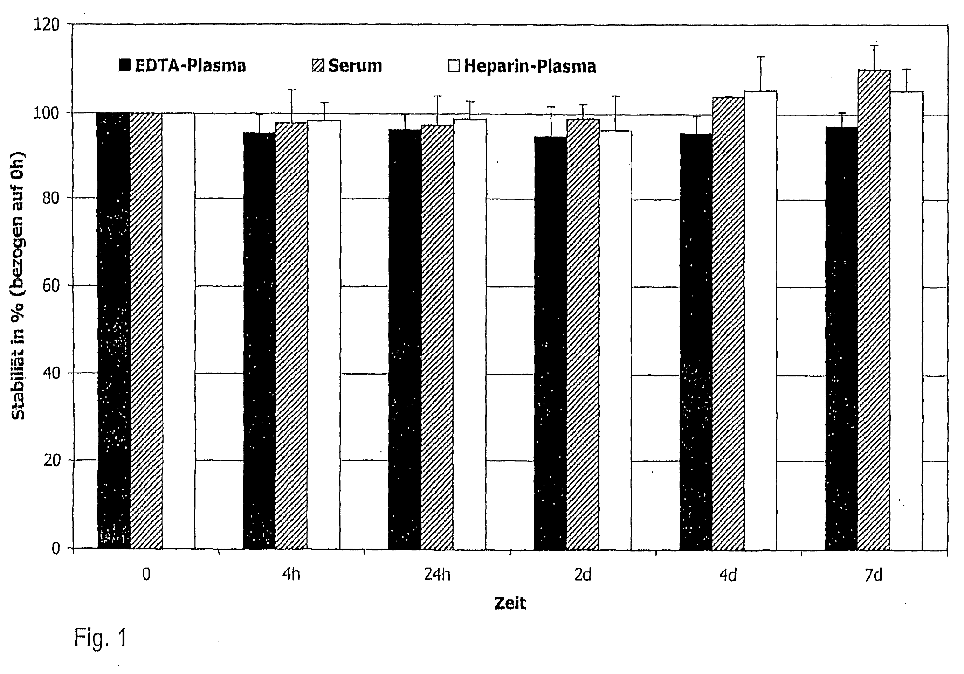

Die Ergebnisse der Stabilitätsmessungen sind in Figur 1 gezeigt.

Somit ist die Bestimmung von Proneurotensin bzw. PNT-Fragmenten in Blutproben voll routinetauglich und geeignet, die NT/NMN/PNT-Freisetzungsrate im Blut der medizinischen Routinediagnostik zugänglich zu machen.

Die Herstellung des verwendeten Assays, sein Verwendung bei Messungen sowie die mit ihm erhaltenen Messergebnisse werden im nachfolgenden experimentellen Teil und den Beispielen und den zugehörigen Erläuterungen noch genauer beschrieben.

### Experimenteller Teil:

Bestimmung einer N-terminalen Proneurotensinsequenzen zuzuordnenden Immunreaktivität im Plasma

### 1. Herstellung von Antikörpern

### 1.1. Immunogene

Es wurden 2 verschiedene Peptid-Teilsequenzen des humanen Preproneurotensins (SEQ ID NO:1), nämlich PNT3 (SEQ ID NO:4) bzw. PNT4 (SEQ ID NO:5), ausgewählt und von der Firma Jerini (Berlin, Deutschland) als synthetische Peptide synthetisiert. Jedes Peptid wurde zusätzlich mit einem aminoterminalen Cysteinrest (Cys0) versehen.

### 1.2. Antikörpergewinnung

Für Immunisierungszwecke wurden die durch N-terminale Cystein-Reste ergänzten Peptide PNT3 (SEQ ID NO:4) und PNT4 (SEQ ID NO:5) mit dem Hämocyanin aus *Limulus polyphemus* konjugiert und zur Immunisierung von Kaninchen nach Standardverfahren verwendet, von denen Antiseren gegen die PNT-Peptid-Konjugate gewonnen wurden.

### 1.3. Reinigung der Antikörper

Die polyklonalen Antikörper der Kaninchen-Antiseren wurden mittels ligandenspezifischer Affinitätsreinigung aufgereinigt. Hierfür wurden die Cys(0)-Peptide PNT3 (SEQ ID NO:4) bzw. PNT4 (SEQ ID NO:5) zunächst an SulfoLink-Gel der Firma Pierce (Boston, USA) gekoppelt. Die Bindung erfolgte nach der Vorschrift des Herstellers wie folgt:

Polycarbonat-Säulen (15mm x 80mm) wurden mit 5ml Affinitätsmatrix befüllt. Nach Äquilibrierung der Säulen mit PBS (136mM NaCl, 1,5mM KH₂PO₄, 20,4mM Na₂HPO₄*2H₂O, 2,7mM KCl, pH 7,2) wurden 5 mg der jeweiligen o.g. Peptide abgewogen, in PBS gelöst, auf die verschlossenen Säulen gegeben, und das Gelmaterial wurde durch Schwenken homogenisiert. Nach 15minütiger Inkubation bei Raumtemperatur und Absetzen des Gelmaterials wurden die Säulen mit 5mal 3ml PBS gewaschen. Zur Absättigung freier Bindungsstellen wurden dem Säulenmaterial je 5ml einer 50mM L-Cysteinlösung hinzugegeben und das Gelmaterial wurde nach Homogenisierung erneut bei Raumtemperatur für 15min inkubiert. Nach Absetzen des Gelmaterials wurde jede Säule mit 6mal 5ml einer 1M NaCl-Lösung gespült und anschließend nochmals mit PBS gespült.

Das Gelmaterial wurde mit 25ml des jeweiligen Kaninchen-Antiserumpools gemischt und über Nacht bei Raumtemperatur unter leichtem Schwenken inkubiert. Die Serum-Gel-Gemische wurden in Polycarbonat-Säulen überführt, und überschüssiges Serum wurde entfernt. Die Säulen wurden anschließend mit 250 ml PBS gewaschen, um nicht gebundene Serumproteine zu entfernen. Die Desorption der gebundenen Antikörper erfolgte durch Elution der Säule mit 50mM Citronensäure (pH 2,2). Das Eluat wurde in Fraktionen á 1ml aufgefangen. Die Proteinkonzentration jeder Fraktion wurde mit Hilfe des BCA-Proteinassay-Kits der Firma Perbio (Bonn, Deutschland) bestimmt, und Fraktionen mit einem Proteingehalt > 1mg/ml wurden vereinigt. Die affinitätsgereinigten Antikörper wurden mittels Dialyse in PBS umgepuffert, der Proteingehalt erneut bestimmt. Anschließend wurde bei 4°C gelagert.

### 2. Assayherstellung

### 2.1. Festphase

Der affinitätsgereinigte polyklonale Kaninchen-Antikörper gegen das Peptid PNT3 wurden auf Polystyrolröhrchen (Startubes, 12mm x 75mm, Firma Greiner, Deutschland) immobilisiert. Hierfür wurde die Antikörperlösung auf eine Proteinkonzentration von 6,7 µg/ml mit PBS verdünnt, und davon wurden 300µl pro Röhrchen pipettiert (entspricht 2µg Antikörper pro Röhrchen). Diese wurden für 20h bei Raumtemperatur inkubiert und anschließend 3mal mit je 4 ml PBS gewaschen. Bis zur weiteren Verwendung wurden die Röhrchen bei 4°C gelagert.

### 2.2. Markierter Antikörper

Der affinitätsgereinigte polyklonale Kaninchen-Antikörper gegen PNT4 (1mg/ml in PBS) wurde mit Acridiniumester-N-Hydroxy-Succinimid (1mg/ml in Acetonitril, Firma InVent, Hennigsdorf Deutschland) lumineszenzmarkiert. Für die Markierung wurden 200µl Antikörper mit 4µl Acridiniumester gemischt, für 20min inkubiert, und freie Acridiniumesterbindungen wurden durch Zugabe von 40µl einer 50mM Glycin-Lösung abgesättigt. Der Markierungsansatz wurde mittels HPLC an einer BioSil 400-Gelfiltrations-Säule (Firma BioRad, München, Deutschland) von freiem Acridiniumester getrennt. Als Laufmittel wurde PBS verwendet.

### 3. Durchführung von PNT-Bestimmungen

### 3.1. Messbedingungen

Pro Antikörper-beschichtetes Röhrchen (2.1) wurden 50µl einer zu untersuchenden Plasma-Probe sowie 150µl Assaypuffer (PBS-Puffer, 10mM EDTA) pipettiert und 16h bei Raumtemperatur inkubiert. Anschließend wurden die Röhrchen 5mal mit je 1ml PBS gewaschen. Zu den Röhrchen wurden dann je 20 ng des markierten Antikörpers (2.1.) (in 100µl PBS-Puffer, 10mM EDTA) zugegeben. Die Röhrchen wurden für 2h bei RT inkubiert, und anschließend wurde ungebundener Tracer-Antikörper durch 5maliges Waschen mit je 1ml PBS entfernt.

Am Röhrchen gebundener markierter Antikörper wurde mittels Lumineszenzmessung in einem handelsüblichen Luminometer (Berthold LB 952T/16) quantifiziert.

### 3.2. Kalibrierung

Um die Konzentrationen bzw. Immunreaktivitäten von Proneurotensin in den zu vermessenden Proben bestimmen zu können, wurden 50 EDTA-Plasmen von augenscheinlich gesunden Menschen auf PNT gescreent, und Plasmen mit den höchsten PNT-Immunreaktivitäten wurden gepoolt. Aus diesem Plasmapool wurden mehrere Verdünnungsstufen mit Pferdeserum (Sigma, Deutschland) hergestellt. Dem höchsten Standard (unverdünntes EDTA-Plasma) wurde eine fiktive Konzentration von 100 Units/ml zugeordnet. In Figur 2 ist eine PNT-Standardkurve gezeigt. Die analytische Sensitivität des Proneurotensin-Assays liegt bei ca. 1 U/ml.

### 4. Ergebnisse von Messungen

### 4.1. Proneurotensin -Immunreaktivität in Plasmen von augenscheinlich gesunden Menschen

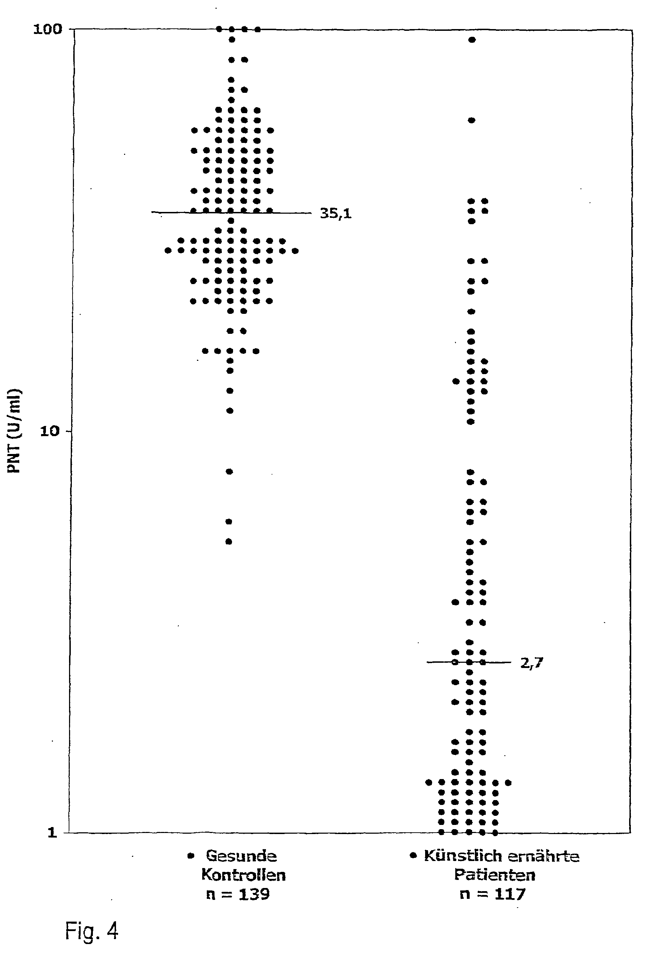

Augenscheinlich gesunde Kontrollpersonen weisen im Blut hohe PNT-Messwerte auf. Es ergibt sich eine Häufigkeitsverteilung, die in Figur 4 gezeigt ist. Der Median wurde mit 35,1 U/ml ermittelt.

### 4.2. Bestimmung der PNT-Immunreaktivität in der Zirkulation von gesunden Probanden vor, während und nach der Nahrungsaufnahme

Es ist bekannt, dass die Bildung bzw. Freisetzung von Neurotensin durch Nahrungsaufnahme stimuliert wird (15; 14). Um zu ermitteln, ob auch die PNT-Konzentration im Blut von der Nahrungsaufnahme beeinflusst wird, und ob die Dynamik der eventuellen Veränderung der PNT-Immunreaktivität parallel zu der vorbeschriebenen von NT verläuft, wurde folgender Versuch durchgeführt: 6 Probanden (je drei männliche und weibliche Personen) fasteten für 14h. Dann wurde ihnen eine Blutprobe entnommen. Die Probanden nahmen dann zu vorgegebenen Zeitpunkten eine definierte Menge an Flüssigkeit bzw. Nahrung zu sich, und zu bestimmten Zeitpunkten wurden erneut Blutproben genommen wurden. Der Ablauf des Versuchs ist in Tabelle 1 zusammengefasst.

**Tabelle 1: Ablauf des Nahrungsstimulationsversuchs**

| Abnahmepunkt | Ernährungsstatus |
|---|---|
| 1 | nach 14h Fasten |
| 2 | nach 15h Fasten |
| 3 | 5 min nach Flüssigkeitsaufnahme (11 Wasser) |
| 4 | 15 min nach Flüssigkeitsaufnahme |
| 5 | 45 min nach Flüssigkeitsaufnahme |
| 6 | 1h 15 min nach Flüssigkeitsaufnahme |
| 7 | 1h 45 min nach Flüssigkeitsaufnahme |
| 8 | 3h nach Flüssigkeitsaufnahme |
| 9 | 5 min nach Nahrungsaufnahme (Salat mit ca. 250 kcal und Pizza mit ca. 100 kcal) |
| 10 | 15 min nach Nahrungsaufnahme |
| 11 | 30 min nach Nahrungsaufnahme |
| 12 | 1h nach Nahrungsaufnahme |
| 13 | 2h nach Nahrungsaufnahme |
| 14 | 3h nach Nahrungsaufnahme |
| 15 | 4h nach Nahrungsaufnahme |

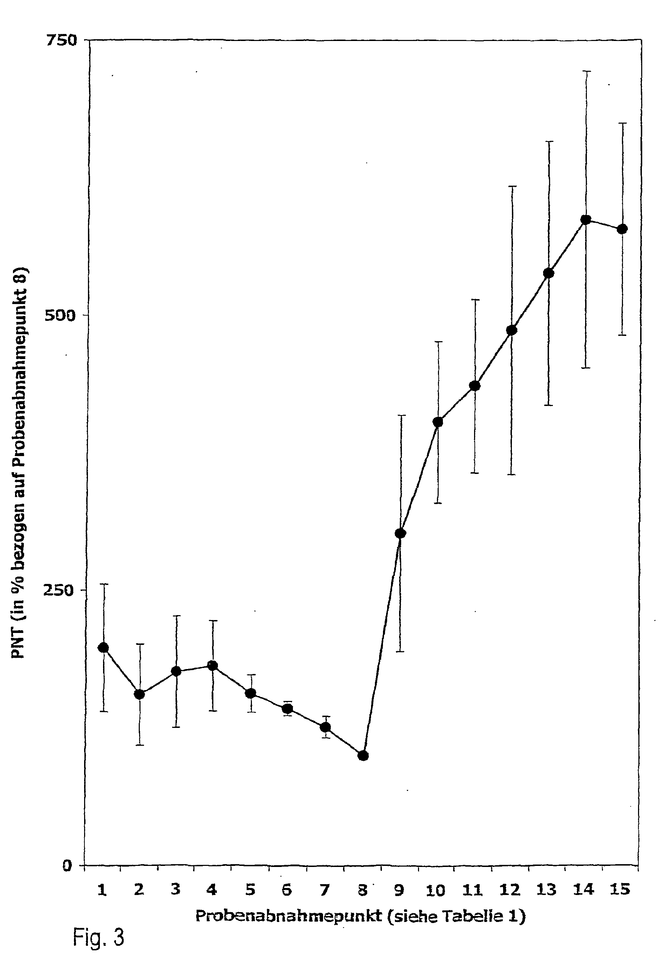

Es zeigt sich, dass die Ausschüttung von PNT durch Aufnahme von Flüssigkeit nicht stimuliert wird. Kurz vor der Nahrungsaufnahme, also nach der längsten Fastenzeit, ist die PNT-Konzentration am geringsten. Sie wurde für jeden einzelnen Probanden als 100% definiert, und alle anderen Messpunkte des jeweiligen Probanden wurden auf diesen Wert bezogen. Diese prozentualen Veränderungen der gemessenen PNT-Immunreaktivität der 6 Probanden wurden gemittelt und sind in Figur 3 graphisch dargestellt (P<0,05). Sofort nach dem Essen steigt die PNT-konzentration im Blut signifikant an. Die letzten beiden Abnahmezeitpunkte 3h und 4h nach der Nahrungsaufnahme weisen die höchsten Messwerte auf. Insgesamt steigt die PNT-Konzentration nach dem Essen um das 4,5- bis 8fache an.

Mit dem o.g. Assay bestimmbare Immunreaktivität, die einem oder mehreren N-terminalen Proneurotensinfragment(en) mit Bindungsstellen für beide verwendeten Antikörper zuzuordnen ist, nimmt somit, wie für das reife Peptid NT beschrieben, aufgrund von Stimulation durch Nahrungsaufnahme zu.

Die Messung von PNT-Immunreaktivität kann somit anstelle des reifen Neurotensins für die Bewertung des metabolischen Status eines Patienten/einer Testperson herangezogen werden. Durch Gabe einer definierten Menge an Nährstoffen kann die individuelle Stimulierbarkeit der PNT-Freisetzung als Unterschied Δ eines Messwerts zu einem vorgegebenen Zeitpunkt nach der Nahrungsaufnahme und einem Basiswert (z.B. PNT₃ₕ nach Nahrungsaufnahme und PNT_{nüchtern}) oder als Verhältnis bzw. Faktor solcher Werte bestimmt werden. Tabelle 2 zeigt beispielhaft eine entsprechende Auswertung.

**Tabelle 2: PNT-Immunreaktivitäten (in U/ml) vor und nach Nahrungsstimulation sowie Angabe von absoluten Differenzen und Faktoren**

| | Proband 1 | Proband 2 | Proband 3 | Proband 4 | Proband 5 | Proband 6 |
|---|---|---|---|---|---|---|
| PNT_{B} | 10,5 | 2,3 | 4,8 | 5,0 | 5,7 | 5,8 |
| PNT₅ₘᵢₙ | 32,3 | 10,7 | 9,6 | 23,0 | 27,1 | 15,5 |
| PNT₃ₕ | 42,4 | 18,4 | 20,0 | 22,7 | 41,5 | 40,6 |
| Δ PNT₅ₘᵢₙ minus PNT_{B} | 21,8 | 8,45 | 4,8 | 18,0 | 21,4 | 9,7 |
| Δ PNT₃ₕ minus PNT_{B} | 31,9 | 16,1 | 15,2 | 17,7 | 35,8 | 34,8 |
| PNT₅ₘᵢₙ / PNT_{B} | 3,1 | 4,7 | 2 | 4 , 6 | 4,8 | 2,7 |
| PNT₃ₕ / PNT_{B} | 4,0 | 8 | 4,2 | 4,5 | 7,3 | 7 |

| | | | | | | |
|---|---|---|---|---|---|---|
| In der Tabelle bedeuten: PNT_{B} = PNT-Immunreaktivität nach 18 h Fasten (Basiswert) PNT₅ₘᵢₙ = PNT-Immunreaktivität 5 min nach Nahrungsaufnahme PNT₃ₕ = PNT-Immunreaktivität 3 h nach Nahrungsaufnahme | | | | | | |

### 4.3. Bestimmung der PNT-Konzentrationen in der Zirkulation von künstlich ernährten Patienten

Wie bereits gezeigt, weisen gesunde Personen eine gewisse PNT-Konzentration im Blut auf, die sich durch Nahrungsaufnahme stimulieren lässt. Bei einer entsprechden Messung der PNT-Immunreaktivität in Plasmen von parenteral (mittels Venenkatheter) künstlich ernährten, zumeist intensivpflichtigen Patienten (z.B. mit einer bestehenden Sepsis oder einem Polytrauma) wurde gefunden, dass diese signifikant niedrigere PNT-Konzentrationen aufweisen als normal ernährte Kontrollen. Bei derartigen Messungen konnte eine um den Faktor 13 verringerte mediane relative PNT-Immunreaktivität von 2,7 U/ml festgestellt werden (siehe Figur 4).

### 4.4 Bestimmung der PNT-Immunreaktivität in der Zirkulation von Patienten mit inflammatorischen Darmerkrankungen (Morbus Crohn bzw. Colitis ulcerosa)

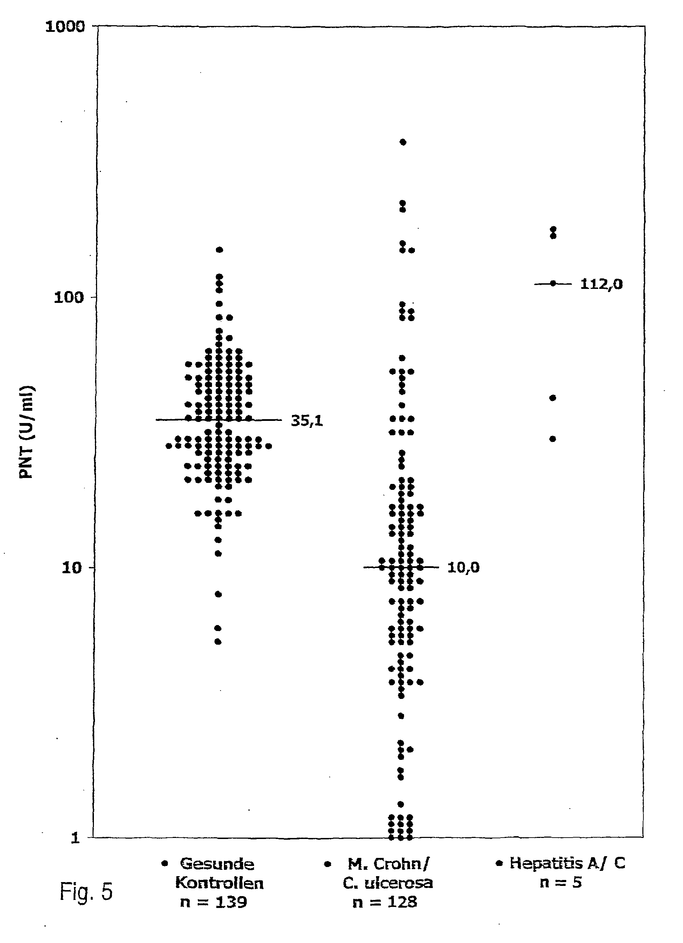

Bei der Messung von Proben von Patienten mit einer inflammatorischen Darmerkrankung, wie Morbus Crohn und Colitis ulcerosa, zeigte sich, dass auch hier die mediane messbare PNT-Immunreaktivität mit 10 U/ml unter der der Kontrollpersonen liegt (Figur 5). Einzelne der Patienten wiesen jedoch auch deutlich höhere Messwerte (über 100 U/ml) auf.

### 4.5 Bestimmung der PNT-Immunreaktivität in der Zirkulation von Patienten mit einer Hepatits A bzw. C

Bei der Messung von Proben von Patienten mit einer Hepatitis A oder C wurden dagegen im Vergleich zu den gesunden Kontrollen höhere PNT-Konzentrationen in der Zirkulation gefunden, mit einem Median von 112 U/ml (Figur 5).

### 5. Isolierung und Charakterisierung von Proneurotensin-Immunreaktivität in Human-Serum

### 5.1. Herstellung der Affinitätssäule

5mg des aufgereinigten polyklonalen Kaninchen-Antikörpers gegen das PNT3-Peptid (SEQ ID NO:4; vgl. 1.1.) wurden nach Vorschrift des Herstellers an 2ml CarboLink-Gel (Firma Pierce, Boston, USA) gekoppelt und in eine Polycarbonatsäule (15mm x 80mm) überführt. Anschliessend wurde das Gel mit 20ml PBS gewaschen.

### 5.2. Affinitätsreinigung von PNT-Immunreaktivität

Eine Serum-Mischprobe aus 10 individuellen Seren á 100ml mit einer relativen Konzentration von 32 U/ml wurde mit Na-EDTA versetzt (Endkonzentration 10mM) und anschliessend über einen 0,2µm-Filter filtriert. Die aufbereitete Serum-Mischprobe wurde bei 4°C und einer Durchflussrate von 2ml/min sechsmal hintereinander über die Affinitätssäule gepumpt. Anschliessend wurde die Säule mit 50ml PBS gewaschen und die gebundene PNT-Immunreaktivität mit einer 50mM Glycin/ HCl-Lösung (pH 2,0) eluiert. Der Säulenausfluß wurde kontinuierlich auf Absorption bei 280nm überwacht und die von der Glycin/HCl-Lösung eluierte Proteinfraktion (Endvolumen 3ml) wurde weiter mittels HPLC analysiert.

### 5.3. Reversed-Phase-HPLC-Analyse

Das durch Affinitätsreinigung gewonnene Material wurde über Reversed-Phase-HPLC an einer C₁₈-Säule µ Bondapak 0,4x30mm der Firma Waters (Eschborn, Deutschland) gereingt. Die Durchflussgeschwindigkeit betrug 1ml/min. Die verwendeten Laufmittel und Elutionsbedingungen sind in der folgenden Tabelle 3 angegeben.

**Tabelle 3: Elutionsbedingungen der RP-HPLC von Proneurotensin-IR**

| | | |
|---|---|---|
| Laufmittel A: 5% Acetonitril 20 mM NH₄-Acetat | | |
| Laufmittel B: 90% Acetonitril 20 mM NH₄-Acetat | | |
| Gradient | 0,0 min | 100% A / 0% B |
| | 2,5 min | 100% A / 0% B |
| | 5,0 min | 89% A / 11% B |
| | 55,0 min | 30% A / 70% B |
| | 60,0 min | 0% A / 100% B |

Der Säulenausfluss wurde kontinuierlich auf seine Absorption bei 214nm vermessen und in Fraktionen von 0,5ml gesammelt. Mit Hilfe des in den obigen Abschnitten 2. und 3. beschriebenen PNT-Assays wurden diejenigen Fraktionen bestimmt, in denen eine PNT-Immunreaktivität nachweisbar war. Es zeigte sich, dass die Haupt-Immunreaktivität in der Fraktion 67 eluiert wurde.

Die Fraktion 67, die eine positive PNT-Immunreaktivität aufwies, wurde durch Begasen mit Stickstoff getrocknet. Anschliessend wurden die Proben massenspektrometrisch analysiert.

### 5.4. Massenspektrometrische Analyse

Das Peptid wurde mittels Protease Glu-C bzw. Trypsin verdaut, die entstandenen Fragmente wurden auf an sich bekannte Weise über SMART-HPLC gewonnen und anschliessend massenspektrometrisch untersucht.

Bei der massenspektrometischen Analyse wurde eine Sequenz (SEQ ID NO:6) ermittelt, die vollständig mit der Sequenz der Aminosäuren 24 - 140 des bekannten Preproneurotensins 1-170 (SEQ ID NO:1) übereinstimmte. Damit wurde nachgewiesen, dass im Blut von Menschen ein Proneurotensin-Peptid zirkuliert, das 117 Aminosäuren umfasst und als Preproneurotensin 24-140 zu bezeichnen ist. Es ergab sich keinerlei Hinweis darauf, dass die mittels des beschriebenen Verfahrens als Immunreaktivität bestimmte Spezies noch die C-terminalen reifen Peptide Neuromedin N und Neurotensin enthält.

### Literatur

1. ALLEN J. M., PENKETH A. R., ADRIAN T. E., LEE Y. C., SARSON D. L., HODSON M. E., BATTEN J. C., BLOOM S. R. (1983). Adult cystic fibrosis: postprandial response of gut regulatory peptides. Gastroenterology 85: 1379-1383
2. BARDELLA M. T., FRAQUELLI M., PERACCHI M., CESANA B. M., BIANCHI P. A., CONTE D. (2000). Gastric emptying and plasma neurotensin levels in untreated celiac patients. Scandinavian Journal of Gastroenterology 35:269-73
3. BARELLI H., FOX-THRELKELD J. E., DIVE V., DANIEL E. E., VINCENT J. P., CHECLER F. (1994). Role of endopeptidase 3.4.24.16 in the catabolism of neurotensin, in vivo, in vascularly perfused dog ileum. British Journal of Pharmacology 112: 127 + 132
4. BECK B. (2000). Neuropeptides and obesity. Nutrition 16: 916-923
5. BEINFELD M. C. (1998). Prohormone and proneuropeptide processing. Recent progress and future challenges. Endocrine 8:1-5
6. BISSETTE G., NEMEROFF C. B., LOOSEN P. T., PRANGE A. J. JR., LIPTON M. A. (1976). Hypothermia and intolerance to cold induced by intracisternal administration of the hypothalamic peptide neurotensin. Nature 262:607-9
7. CARRAWAY R. E., MITRA S. P., SPAULDING G. (1992). Posttranslational processing of the neurotensin/neuromedin-N precursor. Annals of the New York Academy of Sciences 668:1-16
8. CLINESCHMIDT B. V., MARTIN G. E., VEBER D. F. (1982). Antinocisponsive effects of neurotensin and neurotensinrelated peptides. Annals of N Y Acadademy Sciences 400:283-306
9. DOBNER P. R., BARBER D. L., VILLA-KOMAROFF L., MCKIER-NAN C. (1987). Cloning and sequence analysis of cDNA for the canine neurotensin/neuromedin N precursor. PNAS USA 84: 3516-3520
10. EVERS B. M., IZUKURA M., CHUNG D. H., PAREKH D., YOS-HINAGA K., GREELEY G. H. JR., UCHIDA T., TOWNSEND C. M. JR., THOMPSON J. C. (1992). Neurotensin stimulates growth of colonic mucosa in young and aged rats. Gastroenterology 103:86-91
11. FERRARO L., TANGANELLI S., O'CONNER W. T., BIANCHI C., UNGERSTEDT U., FUXE K. (1995). Neurotensin increases endogenous glutamate release in the neostriatum of the awake rat. Synapse 20: 362-364
12. FEURLE G. E., MULLER B., RIX E. (1987). Neurotensin induces hyperplasia of the pancreas and growth of the gastric antrum in rats. Gut 28 Suppl:19-23
13. FRIRY C., FELICIANGELI S., RICHARD F., KITABGI P., ROVERE C. (2002). Production of recombinant large proneurotensin/neuromedin N-derived peptides and characterization of their binding and biological activity. Biochemical and Biophysical Research Communications 290: 1161-1168
14. GULLO L., PEZZILLI R., TOMASSETTI P., DE GIORGIO R. (1998). Plasma cholecystokinin and neurotensin after an ordinary meal in humans. A prolonged study. Gastroenterological and Clinical Biology 22: 25-28
15. HAMMER R. A., CARRAWAY R. E., LEEMAN S. E. (1982). Elevation of plasma neurotensinlike immunoreactivity after a meal. Journal of Clinical Investigation 70: 74-81
16. HERZIG K. H., DOMAGK J., NUSTEDE R., STERN M., BEWIG B., CICHY W. K., FOLSCH U. R. (1997). Plasma concentrations of cholecystokinin and neurotensin in patients with cystic fibrosis. Scandinavian Journal of Gastroenterology 32: 315-319
17. KISLAUSKIS E., BULLOCK B., MCNEIL S., DOBNER P. R. (1988). The rat gene encoding neurotensin and neuromedin N. Structure, tissue-specific expression, and evolution of exon sequences. Journal Biological Chemistry 263: 4963-4968
18. KITABGI P. (1989). Neurotensin modulates dopamine transmission at several levels along brain dopaminergic pathways. Neurochemistry International 14: 111-119
19. LAPCHAK P., ARAUJO D. M., QUIRION R., BEAUDET A. (1990). Neurotensin regulation of endogenous acetylcholine release for rat cerebral cortex: effect of quinolinic acid lesions of the basal forebrain. Journal of Neurochemistry 55: 1397-1403
20. LEE Y. C., UTTENTHAL L. O., SMITH H. A., BLOOM S. R. (1986). In vitro degradation of neurotensin in human plasma. Peptides 7: 383-387
21. MURPHY M. S., BRUNETTO A. L., PEARSON A. D., GHATEI M. A., NELSON R., EASTHAM E. J., BLOOM S. R., GREEN A. A. (1992). Gut hormones and gastrointestinal motility in children with cystic fibrosis. Digestive Diseases and Sciences 37: 187-192
22. NUSTEDE R., KOHLER H., FOLSCH U. R., SCHAFMAYER A. (1991). Plasma concentrations of neurotensin and CCK in patients with chronic pancreatits with and without enzyme substitution. Pancreas 6: 260-265
23. REINECKE M., WEIHE E., CARRAWAY R. E., LEEMAN S. E., FORSSMANN W. G. (1982). Localization of neurotensin immunoreactive nerve fibres in the guinea-pig heart: evidence derived by immunohistochemistry, radioimmunoassay and chromatography. Neuroscience 7: 1785-1795
24. ROKAEUS A., FRIED G., LUNDBERG J. M. (1984). Occurrence, storage and release of neurotensin-like immunoreactivity from the adrenal gland. Acta Physiology Scandinavia 120: 373-80
25. ROVERE C., BARBERO P., MAORET J.-J., LABURTHE M., KITABGI P. (1998). Pro-neurotensin/neuromedin N expressiion and processing in human colon cancer cell lines. Biochemical and Biophysical Research Communications 246: 155-159
26. RÖKAEUS A., THOR K., ROSELL S. (1984). Cigarette smoking potentiates fat-induced elevation of neurotensin-like immunoreactivity in human plasma. Acta Physiologica Scandinavia 121: 181-184
27. ROSELL S., RÖKAEUS A. (1979). The effect of ingestion of amino acids, glucose and fat on circulating neurotensin-like immunreactivity. Acta Physiologica Scandinavia 107: 263-267
28. ROSTENE W., BROUARD A., DANA C., MASUO Y., AGID F., VIAL M., LHIAUBET A. M., PELAPRAT D. (1992). Interaction between neurotensin and dopamine in the brain. Morphofunctional and clinical evidence. Annals N Y Academy Sciences 668:217-31
29. ROWE W, VIAU V, MEANEY MJ, QUIRION R. (1992). Central administration of neurotensin stimulates hypothalamic-pituitary-adrenal activity. The paraventricular CRF neuron as a critical site of action. Annals N Y Academy Sciences 668:365-7
30. SCHIMPFF R.-M., AVARD C., FENELON G., LHIAUBET A.-M., TENNEZE L., VIDAILHET M., ROSTENE W. (2001). Increased plasma neurotensin concentrations in patients with parkinson's disease. Journal of Neurological and Neurosurgical Psychiatry 70: 784-786
31. SCHIMPFF R. M., GOURMELEN M., SCARCERIAUX V., LHIAUBET A. M., ROSTENE W. (1995). Plasma neurotensin levels in humans: relation to hormone levels in diseases involving the hypothalamo-pituitary-thyroid axis. European Journal of Endocrinology 133: 534-538
32. STROUD RM, WALTER P. (1999). Signal sequence recognition and protein targeting. Current Opinion Structure Biology 9:754-9
33. VINCENT J. P. (1995). Neurotensin receptors: binding properties, transduction pathways, and structure. Cellular Molecular Neurobiology 15:501-12
34. WILDING J. P. (2002). Neuropeptides and appetite control. Diabetic medicine 19: 619-627
35. WISEN O. BJORVELL H., CANTOR P., JOHANNSSON C., THEO-DORSSON E. (1992). Plasma concentrations of regulatory peptides in obesity following modified sham feeding (MSF) and a liquid test meal. Regulatory Peptides 39: 43-54
36. WOOD J. G., HOANG H. D., BUSSJAEGER L. J., SOLOMON T. E. (1988). Neurotensin stimulates growth of small intestine in rats. American Journal of Physiology 255:G813-7.
37. BIDARD JEAN-NOEL et al., Immunological and biochemical characterization of processing products from neurotensin/- neuromedin N precursor in the rat medullary thyroid carcinoma 6-23 cell line. Biochemical Journal, 1993, 291:225-233.
38. EKLUND S et al., Effects of cholera toxin Escherichia coli heat stable toxin and sodium deoxycholate on neurotensin release from the ileum in-vivo, Regulatory Peptides, 29:241-252.

## Patentansprüche

1. Immundiagnostisches Bestimmungsverfahren zur Bestimmung der Freisetzung von Neurotensin in die Säugetier-Zirkulation, **dadurch gekennzeichnet, dass** man in einer Serum- oder Plasmaprobe eines Säugetier-Probanden selektiv eine Immunreaktivität des N-terminalen Teils eines Säugetier-Proneurotensins (PNT-Immunreaktivität) bestimmt, die keine Neurotensin- oder Neuromedin-Immunreaktivität ist.

2. Verfahren nach Anspruch 1, **dadurch gekennzeichnet, dass** man die Immunreaktivität des N-terminalen Teils des PNT in der Serum- oder Plasmaprobe mit Hilfe von Antikörpern bestimmt, die eine spezifische Bindung mit Teilsequenzen des N-terminalen Teil des PNT eingehen.

3. Verfahren nach Anspruch 2, **dadurch gekennzeichnet, dass** man die PNT-Immunreaktivität in der Serum-oder Plasmaprobe unter Verwendung von zwei verschiedenen Antikörpern, die spezifische Bindungen mit unterschiedlichen Teilsequenzen des N-terminalen Teils des PNT eingehen, in einem Sandwichassay bestimmt.

4. Verfahren nach einem der Ansprüche 1 bis 3, **dadurch gekennzeichnet, dass** das Säugetier-Proneurotensin humanes Proneurotensin ist, das in humanem Serum oder Plasma bestimmt wird.

5. Verfahren nach Anspruch 4, **dadurch gekennzeichnet, dass** man selektiv eine PNT-Immunreaktivität bestimmt, die Peptidsequenzen des humanen Preproneurotensins (SEQ ID NO:1) im Bereich der Aminosäuren 24 bis 140 (SEQ ID NO:6) des vollständigen humanen Preproneurotensins zuzuordnen ist.

6. Verfahren nach Anspruch 4 oder 5, **dadurch gekennzeichnet, dass** man die Bestimmung als Sandwichassay mit zwei Antikörpern durchführt, die spezifisch an die Aminosäuresequenzen 67 bis 85 (SEQ ID NO:4) bzw. 121 bis 140 (SEQ ID NO:5) des vollständigen humanen Preproneurotensins (SEQ ID NO:1) binden.

7. Verfahren nach einem der Ansprüche 2 bis 6, **dadurch gekennzeichnet, dass** die Antikörper spezifische monoklonale und/oder affinitätsgereinigte polyklonale Antikörper sind.

8. Verfahren nach einem der Ansprüche 2 bis 7, **dadurch gekennzeichnet, dass** einer der Antikörper durch Immunisierung eines Tiers mit einem Antigen erhalten wird, das eine synthetische Peptidsequenz enthält, die die Aminosäuren 67 bis 85 (SEQ ID NO:4) des humanen Preproneurotens (SEQ ID NO:1) umfaßt, und der andere der Antikörper durch Immunisierung mit einem Antigen erhalten wird, das eine synthetische Peptidsequenz enthält, die die entsprechenden Aminosäuren 121 bis 140 (SEQ ID NO:5) umfaßt.

9. Verfahren nach einem der Ansprüche 3 bis 8, **dadurch gekennzeichnet, dass** einer der Antikörper in festphasengebundener Form eingesetzt wird, und der andere Antikörper markiert ist oder selektiv markierbar ist.

10. Verfahren nach Anspruch 9, **dadurch gekennzeichnet, dass** zur Markierung des markierten oder zu markierenden Antikörpers eine Markierung mit einem Radiosiotop, einem Chemilumineszenz-, Biolumineszenz-, Fluoreszenz- oder Enzymlabel zur Anwendung kommt.

11. Verfahren nach einem der Ansprüche 3 bis 8, **dadurch gekennzeichnet, dass** sowohl der erste als auch der zweite Antikörper in einer flüssigen Reaktionsmischung dispergiert eingesetzt werden und daß an den ersten Antikörper eine erste Markierungskomponente gebunden ist, die Teil eines auf Fluoreszenz- oder Chemilumineszenz-Löschung oder -Verstärkung beruhenden Markierungssystems ist, und daß die zweite Markierungskomponente dieses Markierungssystems an den zweiten Antikörper gebunden ist, so dass nach Bindung beider Antikörper an das nachzuweisende Proneurotensin ein messbares Signal erzeugt wird, das eine Detektion der gebildeten Sandwichkomplexe in der Messlösung ermöglicht.

12. Verfahren nach Anspruch 11, **dadurch gekennzeichnet, dass** das Markierungssystem Seltenerdkrypate oder -chelate in Kombination mit einem Fluoreszenz- oder Chemilumineszenz-Farbstoff, insbesondere von Cyanintyp, umfaßt.

13. Verfahren nach einem der Ansprüche 1 bis 9, **dadurch gekennzeichnet, dass** es als Point-of-Care-Verfahren unter Verwendung einer immunchromatographischen Messvorrichtung durchgeführt wird und als Label zur Markierung ein direkt visuell detektierbares Label verwendet wird.

14. Verfahren nach einem der Ansprüche 1 bis 13, **dadurch gekennzeichnet, dass** es zur Überwachung des Ernährungsstatus und/oder der Verdauungsfunktionen eines Patienten durchgeführt wird.

15. Verfahren nach Anspruch 14, **dadurch gekennzeichnet, dass** die Patienten künstlich ernährte Patienten sind.

16. Verfahren nach einem der Ansprüche 1 bis 15, **dadurch gekennzeichnet, dass** die Bestimmung im Zusammenhang mit der Diagnose, Verlaufskontrolle oder Therapie unter Einsatz von Proben von Patienten erfolgt, die an Erkrankungen leiden, die ausgewählt sind aus Morbus Parkinson, zöliakie, chronisch-entzündlichen Darmerkrankungen (Morbus Crohn oder Colitis ulcerosa), Darmkrebs oder Krebs der Bauchspeicheldrüse, Pankreatitis, Schilddrüsenerkrankungen, cystischer Fibrose und Hepatitis.

## Claims

1. An immunodiagnostic assay method for determining the release of neurotensin into the mammalian circulation, **characterized in that** an immunoreactivity of the N-terminal portion of a mammalian proneurotensin (PNT immunoreactivity) is selectively determined in a serum sample or plasma sample of a test mammal, said immunoreactivity not being neurotensin or neuromedin immunoreactivity.

2. The method as claimed in claim 1, **characterized in that** the immunoreactivity of the N-terminal portion of the PNT is determined in the serum sample or plasma sample with the aid of antibodies which undergo specific binding to partial sequences of the N-terminal portion of the PNT.

3. The method as claimed in claim 2, **characterized in that** the PNT immunoreactivity in the serum sample or plasma sample is determined in a sandwich assay with the use of two different antibodies which undergo specific binding to different partial sequences of the N-terminal portion of the PNT.

4. The method as claimed in any of claims 1 to 3, **characterized in that** the mammalian proneurotensin is human proneurotensin which is determined in human serum or plasma.

5. The method as claimed in claim 4, **characterized in that** a PNT immunoreactivity which can be assigned to peptide sequences of human preproneurotensin (SEQ ID NO:1) in the region of the amino acids 24 to 140 (SEQ ID NO:6) of the complete human preproneurotensin is selectively determined.

6. The method as claimed in claim 4 or 5, **characterized in that** the determination is carried out as a sandwich assay with two antibodies which bind specifically to the amino acid sequences 67 to 85 (SEQ ID NO: 4) and 121 to 140 (SEQ ID NO:5) of the complete human preproneurotensin (SEQ ID NO:1).

7. The method as claimed in any of claims 2 to 6, **characterized in that** the antibodies are specific monoclonal and/or affinity-purified polyclonal antibodies.

8. The method as claimed in any of claims 2 to 7, **characterized in that** one of the antibodies is obtained by immunization of an animal with an antigen which contains a synthetic peptide sequence which comprises the amino acids 67 to 85 (SEQ ID NO:4) of human preproneurotensin (SEQ ID NO:1), and the other of the antibodies is obtained by immunization with an antigen which contains a synthetic peptide sequence which comprises the corresponding amino acids 121 to 140 (SEQ ID NO:5).

9. The method as claimed in any of claims 3 to 8, **characterized in that** one of the antibodies is used in a form bound to a solid phase, and the other antibody is marked or is selectively markable.

10. The method as claimed in claim 9, **characterized in that** marking with a radioisotope or a chemiluminescence, bioluminescence, fluorescence or enzyme label is used for marking the marked antibody or antibody to be marked.

11. The method as claimed in any of claims 3 to 8, **characterized in that** both the first and the second antibody are used in dispersion in a liquid reaction mixture, and that a first marking component which is part of a marking system based on fluorescence or chemiluminescence extinction or amplification is bound to the first antibody, and that the second marking component of this marking system is bound to the second antibody so that, after binding of both antibodies to the proneurotensin to be detected, the measurable signal is produced which permits detection of the resulting sandwich complexes in the measuring solution.

12. The method as claimed in claim 11, **characterized in that** the marking system comprises rare earth cryptates or chelates in combination with a fluorescent or chemiluminescent dye, in particular of the cyanine type.

13. The method as claimed in any of claims 1 to 9, which is carried out as a point-of-care method with the use of an immunochromatographic measuring apparatus, and a directly visually detectable label is used as a label for marking.

14. The method as claimed in any of claims 1 to 13, which is carried out for monitoring the nutrition status and/or the digestive functions of a patient.

15. The method as claimed in claim 14, **characterized in that** the patients are artificially fed patients.

16. The method as claimed in any of claims 1 to 15, **characterized in that** the determination is effected in the context of diagnosis, monitoring or treatment with the use of samples of patients who are suffering from diseases which are selected from Parkinson's disease, celiac disease, chronic inflammatory bowel diseases (Crohn's disease or Colitis ulcerosa), intestinal cancer or cancer of the pancreas, pancreatitis, thyroid diseases, cystic fibrosis and hepatitis.

## Revendications

1. Procédé d'analyse immunodiagnostique pour la détermination de la libération de la neurotensine dans la circulation d'un mammifère, **caractérisé en ce que** l'on détermine dans un échantillon de sérum ou de plasma d'un sujet mammalien, sélectivement, l'immunoréactivité de la partie N-terminale d'une proneurotensine de mammifère (immunoréactivité de la PNT), qui n'est pas l'immunoréactivité de la neurotensine ou de la neuromédine.

2. Procédé selon la revendication 1, **caractérisé en ce que** l'on détermine l'immunoréactivité de la partie N-terminale de la PNT dans l'échantillon de sérum ou de plasma, à l'aide d'anticorps, qui lient spécifiquement la séquence partielle de la partie N-terminale de la PNT.

3. Procédé selon la revendication 2, **caractérisé en ce que** l'on détermine l'immunoréactivité de la PNT dans l'échantillon de sérum ou de plasma, par un dosage en sandwiche en utilisant deux anticorps différents, qui lient spécifiquement des séquences partielles différentes de la partie N-terminale de la PNT.

4. Procédé selon l'une des revendications 1 à 3, **caractérisé en ce que** la proneurotensine de mammifère est la proneurotensine humaine, qui est déterminée dans le sérum ou plasma humain.

5. Procédé selon la revendication 4, **caractérisé en ce que** l'on détermine sélectivement, une immunoréactivité de la PNT, qui est a rattaché à la séquence peptidique de la préproneurotensine humaine (SEQ ID N°1) au niveau des acides aminés 24 à 140 (SEQ ID N°6) de la préproneurotensine humaine complète.

6. Procédé selon la revendication 4 ou 5, **caractérisé en ce que** l'on réalise la détermination sous forme d'un dosage en sandwiche avec deux anticorps, qui lient spécifiquement les séquences d'acides aminés 67 à 85 (SEQ ID N°4) et respectivement, 121 à 140 (SEQ ID N°5) de la préproneurotensine humaine complète (SEQ ID N°1).

7. Procédé selon l'une des revendications 2 à 6, **caractérisé en ce que** les anticorps sont des anticorps spécifiques monoclonaux at/ou polyclonaux purifiés par affinité.

8. Procédé selon l'une des revendications 2 à 7, **caractérisé en ce qu'**un des anticorps est obtenu par immunisation d'un animal avec un antigène, qui contient une séquence peptidique synthétique, qui comprend les acides aminés 67 à 85 (SEQ ID N°4) de la préproneurotensine humaine (SEQ ID N°1), et que l'autre des anticorps est obtenu par immunisation avec un antigène, qui contient une séquence peptidique synthétique, qui comprend les acides aminés correspondant à 121 à 140 (SEQ ID N°5).

9. Procédé selon l'une des revendications 3 à 8, **caractérisé en ce qu'**un des anticorps est mis en oeuvre sous forme liée à une phase solide, et l'autre anticorps est marqué ou peut être marqué de manière sélective.

10. Procédé selon la revendication 9, **caractérisé en ce que** pour le marquage de l'anticorps marqué ou à marquer, on utilise un marquage avec un radioisotope, un agent chimioluminescent, bioluminescent, fluorescent ou une enzyme.

11. Procédé selon l'une des revendications 3 à 8, **caractérisé en ce que** non seulement le premier, mais également le deuxième anticorps sont mis en oeuvre dispersés dans un mélange réactionnel liquide et que sur le premier anticorps, un premier composant de marquage est lié, qui est une partie d'un système de marquage reposant sur l'extinction ou le renforcement de la fluorescence ou de la chimioluminescence, et que le deuxième composant de marquage de ce système de marquage est lié sur le deuxième anticorps, de sorte qu'après liaison des deux anticorps sur la proneurotensine à détecter, un signal mesurable est produit, qui permet une détection du complexe en sandwiche formé dans la solution de mesure.

12. Procédé selon la revendication 11, **caractérisé en ce que** le système de marquage comprend un kryptate ou chélate de terre rare en combinaison avec un colorant fluorescent ou chimiofluorescent, en particulier de type cyanine.

13. Procédé selon l'une des revendications 1 à 9, **caractérisé en ce qu'**il est réalisé en tant que procédé Point-of-Care en utilisant un dispositif de mesure immunochromatographique et que l'on utilise comme marqueur, un marqueur directement visuellement détectable.

14. Procédé selon l'une des revendications 1 à 13, **caractérisé en ce qu'**il est réalisé pour surveiller l'état nutritionnel et/ou les fonctions de digestion d'un patient.

15. Procédé selon la revendication 14, **caractérisé en ce que** les patients sont des patients artificiellement alimentés.

16. Procédé selon l'une des revendications 1 à 15, **caractérisé en ce que** la détermination est réalisée dans le contexte d'un diagnostic, d'un contrôle de suivi ou d'une thérapie, avec mise en oeuvre d'échantillons de patients, qui souffrent de maladies qui sont choisies parmi la maladie de Parkinson, la maladie coeliaque, des affections intestinales inflammatoires chroniques (maladie de Crohn ou colite ulcéreuse), le cancer de l'intestin ou le cancer du pancréas, la pancréatite, des maladies de la glande thyroïde, la fibrose kystique et l'hépatite.


### SEQUENCE LISTING

<110> SphingoTec GmbH
<120>Immunoassay for the monitoring of neurotensin release in the circulation
<130> 4533 PCT
<150> DE 102005003687.2
   <151> 2005-01-26
<160> 6
<170> PatentIn version 3.2
<210> 1
   <211> 170
   <212> PRT
   <213> Homo Sapiens
<400> 1
   Met Met Ala Gly Met Lys Ile Gln Leu Val Cys Met Leu Leu Leu Ala
   1               5                   10                  15
   Phe Ser Ser Trp Ser Leu Cys Ser Asp Ser Glu Glu Glu Met Lys Ala
               20                  25                  30
   Leu Glu Ala Asp Phe Leu Thr Asn Met His Thr Ser Lys Ile Ser Lys
           35                  40                  45
   Ala His Val Pro Ser Trp Lys Met Thr Leu Leu Asn Val Cys Ser Leu
       50                  55                  60
   Val Asn Asn Leu Asn Ser Pro Ala Glu Glu Thr Gly Glu Val His Glu
   65                  70                  75                  80
   Glu Glu Leu Val Ala Arg Arg Lys Leu Pro Thr Ala Leu Asp Gly Phe
                   85                  90                  95
   Ser Leu Glu Ala Met Leu Thr Ile Tyr Gln Leu His Lys Ile Cys His
               100                 105                 110
   Ser Arg Ala Phe Gln His Trp Glu Leu Ile Gln Glu Asp Ile Leu Asp
           115                 120                 125
   Thr Gly Asn Asp Lys Asn Gly Lys Glu Glu Val Ile Lys Arg Lys Ile
       130                 135                 140
   Pro Tyr Ile Leu Lys Arg Gln Leu Tyr Glu Asn Lys Pro Arg Arg Pro
   145                 150                 155                 160
   Tyr Ile Leu Lys Arg Asp Ser Tyr Tyr Tyr
                   165                 170
<210> 2
   <211> 13
   <212> PRT
   <213> Homo Sapiens
<400> 2
   Gln Leu Tyr Glu Asn Lys Pro Arg Arg Pro Tyr Ile Leu
   1               5                   10
<210> 3
   <211> 6
   <212> PRT
   <213> Homo sapiens
<400> 3
   Lys Ile Pro Tyr Ile Leu
   1 5
<210> 4
   <211> 19
   <212> PRT
   <213> Artificial
<220>
   <223> Synthetic pepitde
<400> 4
   Asn Leu Asn Ser Pro Ala Glu Glu Thr Gly Glu Val His Glu Glu Glu
   1 5 10 15
   Leu Val Ala
<210> 5
   <211> 20
   <212> PRT
   <213> Artificial
<220>
   <223> Synthetic peptide
<400> 5
   Leu Ile Gln Glu Asp Ile Leu Asp Thr Gly Asn Asp Lys Asn Gly Lys
   1 5 10 15
   Glu Glu Val Ile
   20
<210> 6
   <211> 117
   <212> PRT
   <213> Homo sapiens
<400> 6
   Ser Asp Ser Glu Glu Glu Met Lys Ala Leu Glu Ala Asp Phe Leu Thr
   1 5 10 15
   Asn Met His Thr Ser Lys Ile Ser Lys Ala His Val Pro Ser Trp Lys
               20                  25                  30
   Met Thr Leu Leu Asn Val Cys Ser Leu Val Asn Asn Leu Asn Ser Pro
           35                  40                  45
   Ala Glu Glu Thr Gly Glu Val His Glu Glu Glu Leu Val Ala Arg Arg
       50                  55                  60
   Lys Leu Pro Thr Ala Leu Asp Gly Phe Ser Leu Glu Ala Met Leu Thr
   65                  70                  75                  80
   Ile Tyr Gln Leu His Lys Ile Cys His Ser Arg Ala Phe Gln His Trp
                   85                  90                  95
   Glu Leu Ile Gln Glu Asp Ile Leu Asp Thr Gly Asn Asp Lys Asn Gly
               100                 105                 110
   Lys Glu Glu Val Ile
           115